# EUROPEAN PATENT SPECIFICATION

(11) **EP 3 520 720 B1**
(45) Date of publication and mention of the grant of the patent: **29.07.2020**
(21) Application number: 18171898.2
(22) Date of filing: 11.05.2018
(51) Int. Cl.: A61B 17/15, A61B 17/16, A61B 17/17, A61F 2/46

(54) **INSTRUMENTS FOR IMPLANTING BONE INSERTS**
INSTRUMENTE ZUM IMPLANTIEREN VON KNOCHENEINSÄTZEN
INSTRUMENTS PERMETTANT D'IMPLANTER DES INSERTS D'OS

(30) Priority: 06.02.2018 IT 201800002438
(43) Date of publication of application: 07.08.2019
(73) Proprietor: Istituto Ortopedico Rizzoli, 40136 Bologna (IT)
(72) Inventor: FILARDO, Giuseppe, 39100 BOLZANO (IT)
(74) Representative: Conti, Marco

(56) References cited:
- WO-A2-2011/017321
- US-A1- 2009 254 126
- US-A1- 2011 238 068
- US-A1- 2016 213 384

## Description

This invention relates to instruments for implanting bone inserts.

More specifically, this invention relates to instruments for implanting bone inserts in the long bones of the human body.

The application of bone inserts or segments in the long bones is used in modern surgical techniques in order to reconstruct the continuity of the bone following either traumatic events or degeneration, for example due to cancer.

Such reconstructive techniques in many cases allow limb amputation to be avoided and allow recovery of a normal condition and improved quality of life.

In the case of pseudarthrosis or large resections of long bones, for example in the case of tumour removal, it is in fact possible to reconstruct the bone with a massive graft consisting of a bone segment or insert. Such a graft restores the bone anatomically and functionally.

The bone insert used may derive either from a bone from a donor or from an artificial material suitable for the purpose.

For the purposes of this document, bone insert shall mean an insert that is not necessarily made of bone material but which is still compatible with reconstruction of a bone cavity.

Operatively, for example in the case of a pathology of the femur where its continuity is compromised, a suitable cutting instrument is used to resect the problem zone, essentially creating two femur bone stumps, one proximal and the other distal.

In use, by means of the above-mentioned cutting tool (saw) on the ends of the stumps surfaces are created which are intended to make contact with corresponding surfaces of a bone insert to be inserted between the two stumps.

The bone insert is then inserted between the two stumps, after which it is stabilised by means of a metal plate secured to both of the above-mentioned stumps.

Patent document WO2011/017321 provides an example of an instrument for implanting bone inserts between two bone stumps of long bones.

The operating method just described is not free of disadvantages.

A first disadvantage is linked to the fact that, since the above-mentioned surfaces of the stumps are created with manual cuts, it is unlikely that they will be perfectly superposed on those of the insert once the insert has been inserted. Consequently, the system will have some instability, and bone regrowth will be more difficult in the absence of optimum contact.

It is basically a rather unstable equilibrium condition between the two stumps and the insert which, if stressed could tend to abandon its alignment with the stumps, therefore resulting in failure of the operation or in any case delaying healing.

The aim of this invention is to provide instruments for implanting bone inserts which allow the disadvantages of the prior art to be overcome. Another aim of this invention is to provide instruments for implanting bone inserts which are effective and practical to use.

Yet another aim of this invention is to provide instruments which allow the implementing of a technique that is reproducible and precise.

The technical features of the invention, with reference to the above aims, are clearly described in the claims below and its advantages are more apparent from the detailed description which follows, with reference to the accompanying drawings which illustrate a preferred, non-limiting embodiment of the invention by way of example and in which:
- Figure 1 is a schematic perspective top view of components of a preferred embodiment of the instruments made in accordance with this invention;
- Figure 2 is a schematic perspective bottom view of the components of Figure 1;
- Figure 3 is a schematic top plan view of the components of Figure 1;
- Figure 4 is an exploded view of the components of Figure 1;
- Figure 5 is a schematic perspective view of a bone insert in accordance with the instruments according to the invention,
- Figure 6 is a schematic perspective top view of further components of a preferred embodiment of the instruments made in accordance with this invention;
- Figure 7 is a schematic top plan view of the components of Figure 6;
- Figure 8 is a schematic side elevation view of the components of Figure 6;
- Figure 9 is an exploded view of the components of Figure 6;
- Figure 10 is a schematic perspective top view of other components of the preferred embodiment of the instruments in accordance with the invention;
- Figure 11 is an exploded view of the components of Figure 10;
- Figure 12 is a schematic perspective top view of components of an alternative embodiment of the instruments in accordance with this invention;
- Figure 13 is a perspective view from a different angle of the components of Figure 12;
- Figure 14 is a schematic side view of the components of Figures 12 and 13 in different possible configurations for use.

As illustrated in the accompanying figures, the numeral 1 denotes in its entirety a preferred embodiment of the instruments for implanting bone inserts 2 in accordance with this invention.

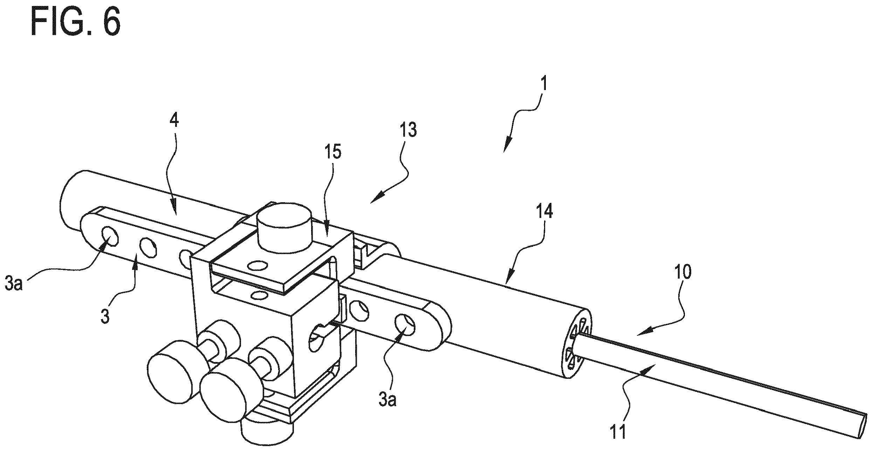

A bone insert of the type implanted with the instruments 1 according to the invention is illustrated in Figure 5.

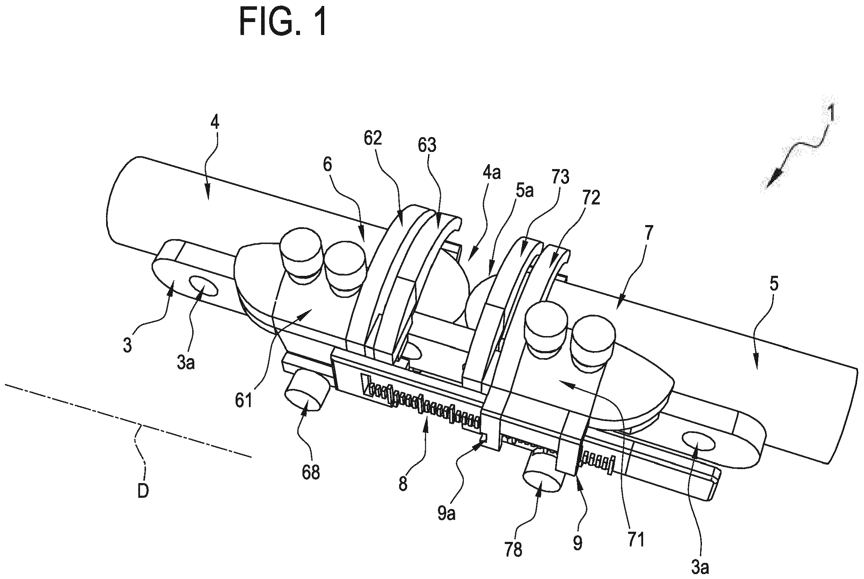

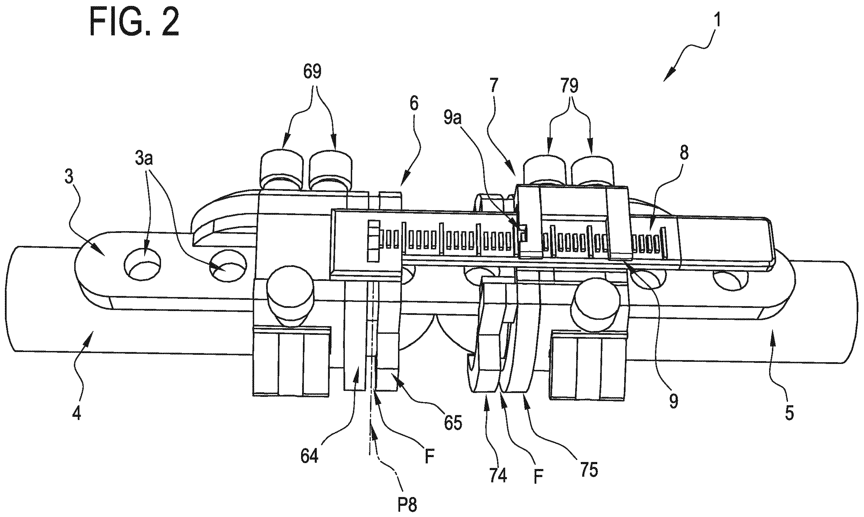

With reference to Figures 1 and 2, a metal plate 3 is provided, extending longitudinally according to a predetermined line D, the plate 3 being suitable for connecting two bone stumps 4, 5; the instruments are used in combination with a metal plate 3, whereby the plate 3 forms part of the instruments for implanting bone inserts 2.

The plate 3 has a plurality of through holes 3a, distributed longitudinally along its predetermined line D of extension.

The holes 3a are suitable for housing respective screws, not illustrated, for fixing the plate 3 to the bone stumps 4, 5.

With reference to Figure 1, the instruments 1 comprise a first and a second guiding unit 6, 7 for cutting respective end portions of the bone stumps 4, 5.

The first guiding unit 6 comprises a central body 61 for engaging with the plate 3 and two upper arms 62, 63 branching from the central body 61 and extending parallel to and spaced from each other.

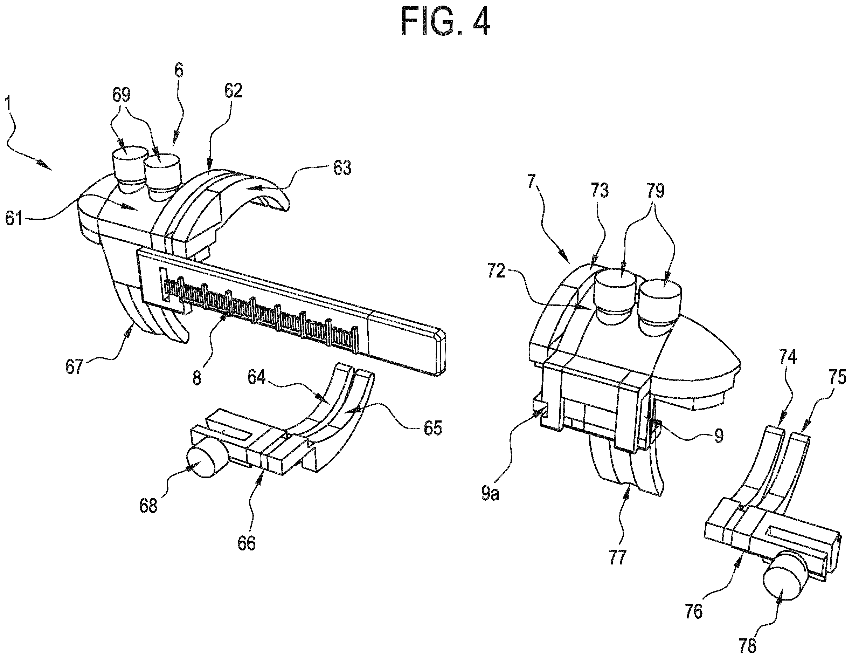

As illustrated in Figure 4, the first guiding unit 6 also comprises two lower arms 64, 65 emerging from a block 66 and suitable for being positioned aligned with the above-mentioned upper arms 62, 63.

The block 66 is slidably fitted on a track 67 fixed to the central body 61. The block 66 supports a clamp screw 68 suitable for securing the block 66 and the arms 64, 65 fixed to it, in a stable position relative to the central body 61.

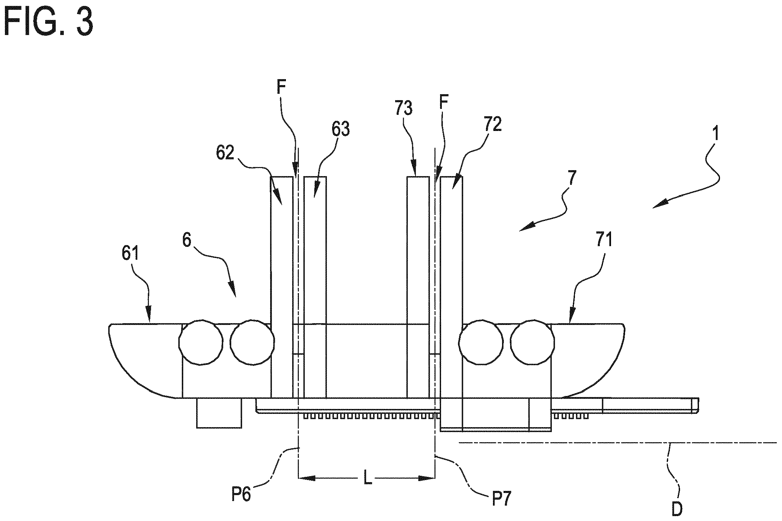

In particular with reference to Figure 3, the space existing between the above-mentioned upper arms 62, 63 and between the above-mentioned lower arms 64, 65 defines a slit F which is configured for guiding a blade, of the known type and not illustrated, for cutting an end portion 4a of the bone stump 4.

In other words, the slit F defines a cutting plane P6, which is sketched in Figure 2, extending perpendicularly to the above-mentioned predetermined line D of extension of the plate 3.

The above-mentioned upper and lower arms 62, 63, 64, 65 extend in a curved fashion by means of which, together with the possibility of adjusting their distance from each other thanks to the slidable block 66, they advantageously allow clasping of a large part of the bone stump 4 to be cut.

Again with reference to Figures 1 to 3, similarly to the first guiding unit 6, the second guiding unit 7 comprises a central body 71 for engaging with the plate 3 and two arms 72, 73 branching from the central body 71 and extending parallel to and spaced from each other.

As illustrated in Figure 4, the second guiding unit 7 also comprises two lower arms 74, 75 emerging from a block 76 and suitable for being positioned aligned with the above-mentioned upper arms 72, 73.

The block 76 is slidably fitted on a track 77 fixed to the central body 71. The block 76 supports a clamp screw 78 suitable for securing the block 76 and the arms 74, 75 fixed to it, in a stable position relative to the central body 71.

In particular with reference to Figure 3, the space existing between the above-mentioned arms 72, 73 defines a slit F which is configured for guiding a blade, of the known type and not illustrated, for cutting an end portion of the bone stump, not illustrated, facing the bone stump 5 visible in the figure.

In other words, the slit F defines a cutting plane P7, which is sketched in Figure 2, extending perpendicularly to the above-mentioned predetermined line D of extension of the plate 3.

The planes P6 and P7 are advantageously parallel to one another.

The above-mentioned upper and lower arms 72, 73, 74, 75 extend in a curved fashion by means of which, together with the possibility of adjusting their distance from each other thanks to the slidable block 76, they advantageously allow clasping of a large part of the bone stump 5 to be cut.

In other words, each of the above-mentioned arms 62, 63, 64, 65, 72, 73, 74, 75 of the first and second guiding units 6, 7 extend in a curved fashion, for approximating the curvature of a cross-section of the long bones and this advantageously allows the surgeon to easily position the cutting blade in the slit F so as to be able to make an accurate, precise cut.

Again with reference to Figures 1 to 3, both the first and second guiding units 6, 7 slidably engage on the plate 3 for moving longitudinally relative to it along the above-mentioned predetermined line D.

For that purpose, on the central body 61, 71 of each guiding unit 6, 7 there is a respective housing, not fully visible in the appended figures, in which the plate 3 slides.

The guiding units 6, 7 comprise respective locking screws 69, 79 for locking the guiding unit 6, 7 relative to the plate 3.

Therefore, the screws 69, 79 can be used to fix the position of the guiding unit 6, 7 relative to the plate 3.

As shown in Figures 1 to 4, the instruments 1 comprise a millimetric graduated ruler 8.

The millimetric graduated ruler 8 is fixed cantilever-style on the central body 61 of the first guiding unit 6 and extends from it towards the second guiding unit 7.

In detail, as in particular illustrated in Figures 1 and 2, the millimetric graduated ruler 8 slidably engages in a pocket 9 made in the central body 71 of the second guiding unit 7, on the pocket 9 there being a reference indicator 9a suitable for indicating the distance of the two guiding units 6, 7, first and second, from each other.

In particular, the distance measured by means of the millimetric graduated ruler 8 is that between the two planes P6 and P7 and labelled L in Figure 3.

The graduated ruler 8 defines, for the instruments 1, respective linear measuring means for measuring the distance between the first and second guiding units 6, 7 which are slidably engaged on the plate 3.

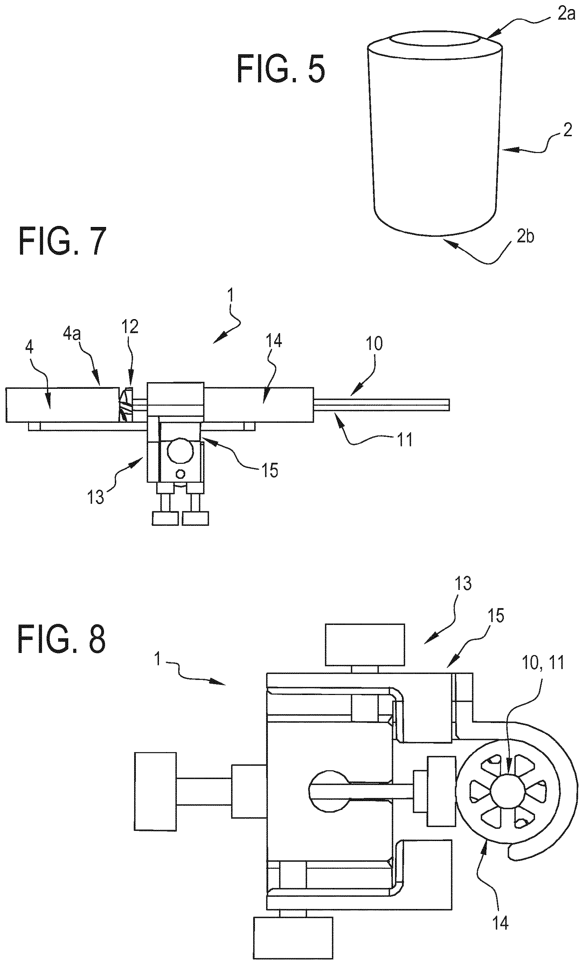

As shown in Figure 6, the instruments 1 comprise a milling cutter 10 for milling the above-mentioned bone stumps 4, 5 at their end portion 4a, 5a. The milling cutter 10 comprises a shaft 11 and an actual milling head 12 which is located at one end of said shaft 11.

Again with reference to Figure 6, the instruments 1 comprise a third guiding unit 13 for supporting and slidably guiding the milling cutter 10. Similarly to the first and second guiding units 6, 7, the third guiding unit 13 is also slidably engaged on the plate 3.

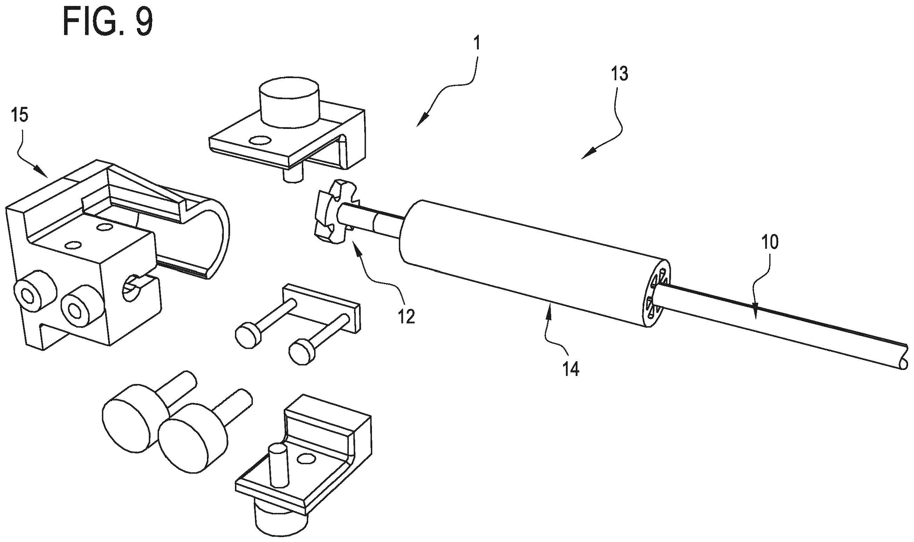

With reference to Figures 6 to 9, the third guiding unit 13 comprises a hollow cylindrical element 14 and a gripping body 15.

The hollow cylindrical element 14 is suitable for slidably housing the shaft 11.

The cylindrical element 14 and the milling head 12 advantageously have the same sized diameter.

The gripping body 15 is suitable for engaging with the plate 3 for stably fixing the third guiding unit 13 to the plate 3.

Advantageously, as shown in Figure 7, the milling head 12 has a frustoconical shape, for making on the above-mentioned end portions 4a, 5a of the stumps 4, 5, respective frustoconical cavities for housing respective frustoconical longitudinal ends 2a, 2b of the insert 2.

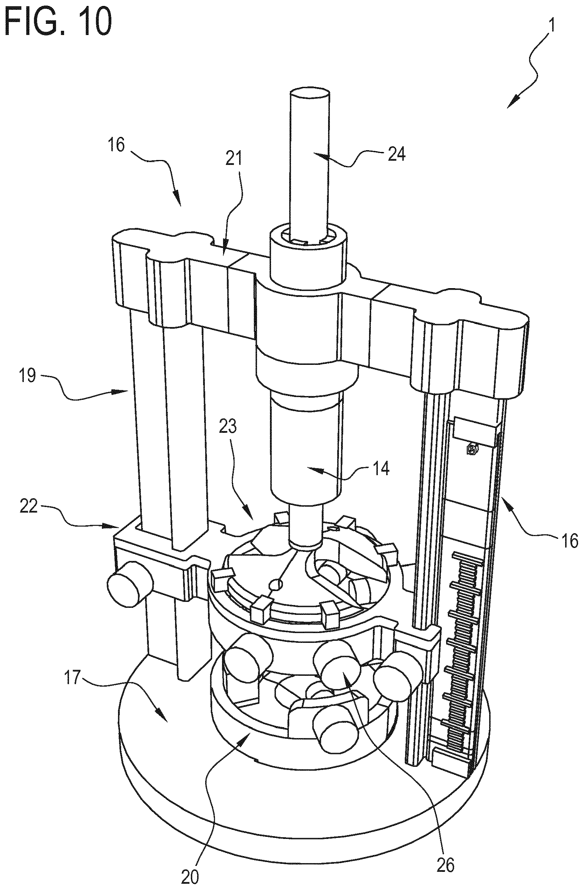

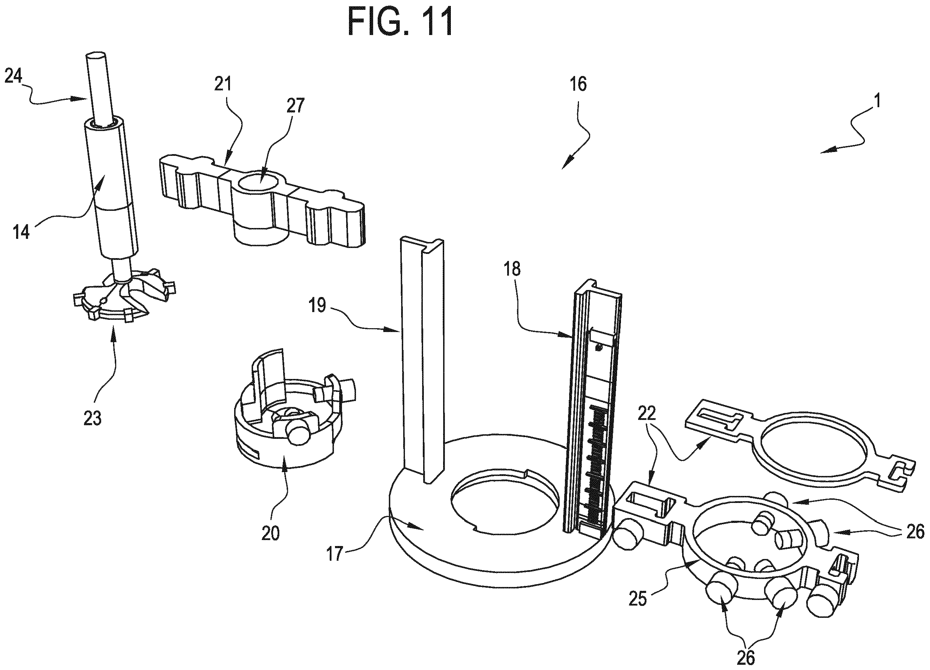

With reference to Figures 10 and 11, the instruments 1 according to the invention comprise a machining device 16 for machining bone inserts 2 to be implanted between the two stumps 4, 5 of long bones.

The machining device 16 comprises a supporting and fixing base 17 for an insert 2 to be machined.

The base 17 has a circular shape and vertically branching from it there two column uprights 18, 19 positioned diametrically opposed to each other. On the base 17, interposed between the two column uprights 18, 19 there is a centring unit 20, suitable for allowing precise positioning of the insert 2 being machined.

At their respective upper ends, the column uprights 18, 19 are connected to each other by means of a first crosspiece 21, which is stationary although it may be detached for assembly.

A second, movable crosspiece 22 is slidably engaged with both of the column uprights 18, 19.

A millimetric graduated scale is made on the upright 18.

The machining device 16 comprises a milling cutter 23.

For the device 16 the milling cutter 23 defines a tool for machining the insert 2.

The milling cutter 23 is shaped so that on the insert 2 is creates respective frustoconical ends 2a, 2b, with the same angle of inclination as the housing of the bone stumps 4, 5 specially prepared.

The milling cutter 23 comprises a respective shaft 24.

On the second crosspiece 22 there is a first circular housing 25 on which the milling cutter 23 abuts during machining of the insert 2.

As shown in Figure 11, along the periphery of the above-mentioned first circular housing 25 there is a plurality of screws 26 for fixing and centring the insert 2, which are suitable for stably holding the insert 2 in the desired position during the machining.

On the first, stationary crosspiece 21 there is a second cylindrical housing 27 in which the shaft 24 of the milling cutter 23 is slidably inserted, directly or by means of an interposed respective hollow cylindrical element 14.

For the machining device 16, the above-mentioned first circular housing 25 and second cylindrical housing 27 define guiding means for the milling cutter 23.

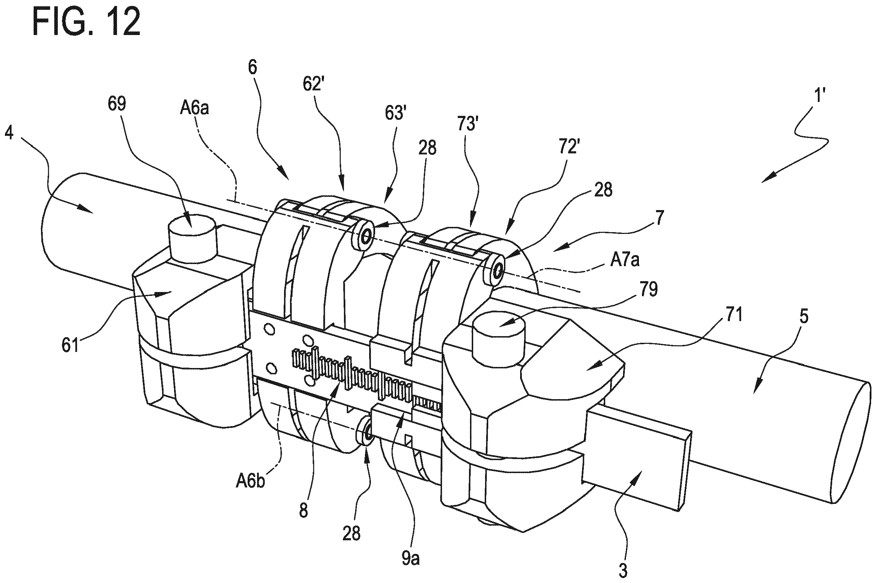

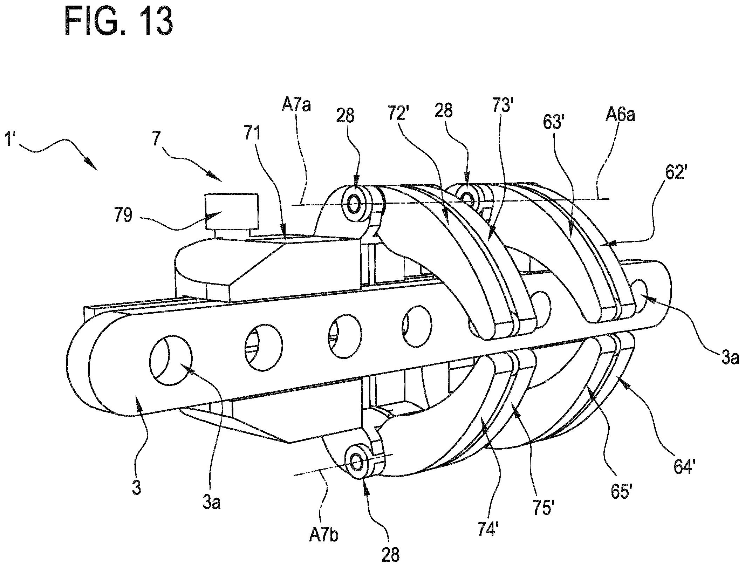

Figures 12 to 14 illustrate an alternative embodiment of the instruments for implanting bone inserts 2 described above reference to Figures 1 to 9. For the sake of practicality, in Figures 12 to 14 corresponding components are labelled with the same reference characters as in the description of the preferred embodiment, to which readers are referred, similarly, for common parts.

With reference to Figures 12 to 14, the alternative embodiment of the instruments illustrated in them is labelled 1' in its entirety.

Unlike the instruments 1 previously described, the instruments 1' comprise respective upper arms 62', 63', 72', 73' and lower arms 64', 65', 74', 75' which are movable relative to the respective central bodies 61, 71.

In detail, the above-mentioned upper arms 62', 63', 72', 73' and lower arms 64', 65', 74', 75' pivot, in pairs, at respective central bodies 61, 71 for oscillating about respective axes A6a, A6b, A7a, A7b in order to get as close as possible to the outer surface of the bone stumps 4, 5 to be treated.

Advantageously, the above-mentioned upper arms 62', 63', 72', 73' and lower arms 64', 65', 74', 75' are rigidly connected, in pairs, in such a way that they rotate together in pairs about the respective axis A6a, A6b, A7a, A7b.

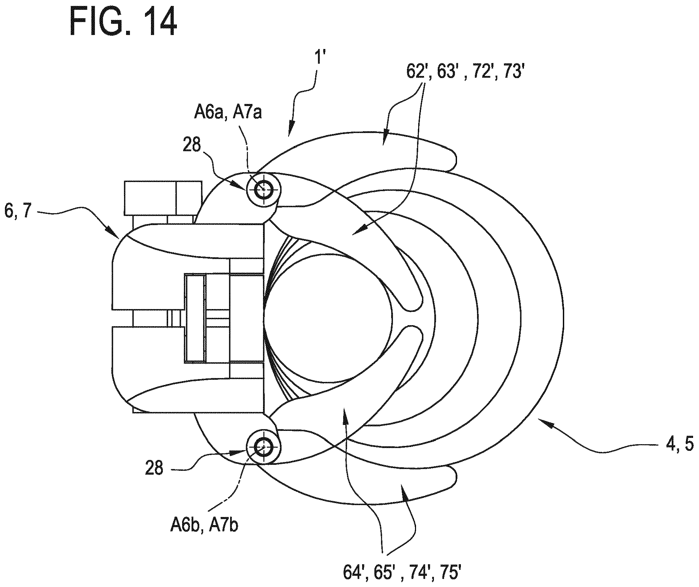

As regards their above-mentioned mobility, Figure 14 reveals various possible configurations that the upper arms 62', 63', 72', 73' and lower arms 64', 65', 74', 75' are able to adopt.

In other words, depending on the actual size (approximated in Figure 14 with a circular dimension) of the bone stumps 4, 5 to which the instruments are applied, the upper arms 62', 63', 72', 73' and lower arms 64', 65', 74', 75' are rotated about the respective axes A6a, A6b, A7a, A7b until they make contact with the outer surface of the stumps 4, 5.

Advantageously, to ensure that the preferred angular position of the upper arms 62', 63', 72', 73' and lower arms 64', 65', 74', 75' is maintained, suitable fixing units are used, for example of the screw type, schematically illustrated in Figures 12 to 14 by a cylindrical element 28. Advantageously, the angle of inclination of the frustoconical surfaces of both the stumps 4, 5 and of the insert 2 is between 10 and 45 degrees. This increases the extent of the contact surfaces, at the same time avoiding excessive angles of inclination which could weaken the tips of the bone stumps 4, 5.

In use, the instruments 1, 1' according to this invention allow precise cuts which are perfectly parallel to each other, of the end portions 4a, 5a of the two bone stumps 4, 5.

For that purpose, the plate 3 is fixed, preferably at its opposite ends and removably, to the two stumps 4, 5 in such a way as to define their ideal alignment condition.

Then one end of the plate is released from the relative stump (for example from the stump 5) in such a way as to free the operating site for operating on a first stump 4.

Installed on the plate 3 there is a first guiding unit 6 and, when the desired position of the cutting plane P6 has been adjusted, cutting of the end 4a of the stump 4 is performed by means of insertion of the above-mentioned cutting blade, not illustrated, in the slit F defined between the respective arms 62, 63 and 64, 65.

When that first cut has been performed, the first guiding unit 6 is removed and, once the two stumps 4, 5 have been realigned, the plate 3 is again fixed to the second stump 5.

Then the end of the plate 3 is released from the relative stump 4, in such a way as to free the operating site for operating on the stump 5.

Similarly to what was done on the stump 4, installed on the plate 3 there is the second guiding unit 7 and, when the desired position of the cutting plane P7 has been adjusted, cutting of the end 5a of the stump 5 is performed by means of insertion of the above-mentioned cutting blade, not illustrated, in the slit F defined by the respective arms 72, 73 and 74, 75. At this point the two stumps have respective facing ends with relative flat surfaces that are parallel to each other.

The second guiding unit is removed and, by installing the gripping body 15 on the plate 3, as illustrated in Figure 6, a step of machining the end of each of the two stumps 4, 5 begins.

In this step, by using the milling cutter 10 slidably inserted in the relative housing made in the gripping body 15, the end 4a of the stump 4 is machined in such a way as to obtain an advantageously frustoconical cavity, shaped to match one end 2a, 2b of the insert 2.

Similarly, after re-alignment of the two stumps 4, 5, detaching of the stump 4 and subsequent positioning of the gripping body 15 near the second bone stump 5, the end 5a of the later is machined, again in order to obtain a cavity that is advantageously frustoconical, shaped to match one end 2a, 2b of the insert 2.

In parallel, as illustrated in Figure 10, the insert 2 is machined by means of the device 16.

In particular, the milling cutter 23 is used to make two frustoconical longitudinal ends 2a, 2b in the insert 2.

In order to be machined, the insert 2 is stably fixed to the base 17 by means of the centring unit 20 and its ends 2a, 2b are machined one after another, by turning it over.

The instruments 1, 1' disclosed achieve the preset aims and bring important advantages.

A first advantage linked to use of the instruments 1, 1' according to the invention is that of obtaining precise, parallel cuts of the end portions of the bone stumps.

Another advantage linked to the invention is that of allowing the obtainment of optimum congruence between the surfaces of the stumps and the insert, thereby improving the equilibrium of the stumps - insert assembly, and also increasing, thanks to the angle of inclination of the frustoconical surfaces, the contact surface area between stump and graft. The increase in the contact surface area between stumps and insert further promotes the mechanical stability and biological potential for synthesis.

## Claims

1. Instruments for implanting bone inserts (2) between two bone stumps (4, 5) of long bones, comprising:
- a metal plate (3) extending longitudinally according to a predetermined direction (D) and suitable for connecting the above-mentioned two bone stumps (4, 5);
- a first guiding unit (6) for cutting an end portion of a first (4) of said two stumps, said first guiding unit (6) being configured for slidably engaging on said metal plate (3) ;
- a second guiding unit (7) for cutting an end portion of a second (5) of said two stumps, said second guiding unit (7) slidably engaging on said plate (3), each of said first and second guiding units (6, 7) having a respective slit (F) for housing and guiding a cutting blade, said slit (F) being configured for guiding a blade as it cuts said end portion of said stumps (4, 5) defining a cutting plane (P6, P7) extending perpendicularly to said predetermined direction (D) of extension of said plate (3).

2. The instruments according to claim 1, **characterised in that** each of said first and second guiding units (6, 7) comprises a central body (61, 71) for engaging with said plate (3) and arms (62, 63, 64, 65, 72, 73, 74, 75) branching from said central body (61, 71) and extending parallel to and spaced from each other, said slit (F) being formed by the space interposed between said arms (62, 63, 64, 65, 72, 73, 74, 75).

3. The instruments according to claim 2, **characterised in that** said arms (62, 63, 64, 65, 72, 73, 74, 75) of said first and second guiding units (6, 7) extend in a curved fashion, for approximating the curvature of a cross-section of the long bones.

4. The instruments according to any one of the preceding claims, **characterised in that** they comprise linear measuring means (8) for the distance between said first and second guiding units (6, 7) which are slidably engaged on said plate (3).

5. The instruments according to claim 4, **characterised in that** said linear measuring means comprise a millimetric graduated ruler (8).

6. The instruments according to any one of the preceding claims, **characterised in that** they comprise a milling cutter (10) for milling one of said bone stumps (4, 5) at its said end portion, a third guiding unit (13) for supporting and slidably guiding said milling cutter (10), said third guiding unit (13) engaging on said plate (3).

7. The instruments according to claim 6, wherein said milling cutter (10) comprises a shaft (11) and a milling head (12) that is located at one end of said shaft (11), **characterised in that** said third guiding unit (13) comprises a hollow cylindrical element (14) suitable for slidably housing said shaft (11), and a gripping body (15) suitable for engaging with said plate (3) for stably fixing said third guiding unit (13) to said plate.

8. The instruments according to claim 6 or 7, **characterised in that** said milling head (12) has a frustoconical shape, for making respective frustoconical cavities on end portions (4a, 5a) of said stumps (4, 5).

9. The instruments according to any one of the preceding claims, comprising a machining device (16) for machining said bone inserts (2) to be implanted between two stumps (4, 5) of long bones, comprising a supporting and fixing base (17) for an insert (2) to be machined, at least one column upright (18, 19) emerging vertically from said base (17), a tool (23) for machining said insert (2) and guiding means (25, 27) for said tool (23) which are supported by said column upright (18, 19).

10. The instruments according to claim 9, **characterised in that** said tool for machining said insert (2) is a milling cutter (23) shaped for making respective frustoconical ends (2a, 2b) on said insert (2).

## Patentansprüche

1. Instrumente zum Implantieren von Knocheneinsätzen (2) zwischen zwei Knochenstümpfen (4, 5) langer Knochen, umfassend:
- eine Metallplatte (3), die sich in Längsrichtung gemäß einer vorbestimmten Richtung (D) erstreckt und zum Verbinden der oben genannten zwei Knochenstümpfe (4, 5) geeignet ist;
- eine erste Führungseinheit (6) zum Schneiden eines Endabschnitts eines ersten (4) der beiden Stümpfe, wobei die erste Führungseinheit (6) konfiguriert ist, um auf die Metallplatte (3) verschiebbar einzugreifen;
- eine zweite Führungseinheit (7) zum Schneiden eines Endabschnitts eines zweiten (5) der beiden Stümpfe, wobei die zweite Führungseinheit (7) auf die Platte (3) verschiebbar eingreift, wobei eine jede der ersten und zweiten Führungseinheiten (6, 7) einen entsprechenden Schlitz (F) zum Aufnehmen und Führen einer Schneidklinge aufweisen, wobei der Schlitz (F) zum Führen einer Klinge konfiguriert ist, während sie den Endabschnitt der Stümpfe (4, 5) schneidet, definierend eine Schnittebene (P6, P7), die sich senkrecht zu der vorbestimmten Richtung (D) der Erstreckung der Platte (3) erstreckt.

2. Instrumente nach Anspruch 1, **dadurch gekennzeichnet, dass** eine jede der ersten und zweiten Führungseinheiten (6, 7) einen zentralen Körper (61, 71) zum Eingreifen mit der Platte (3) und den Armen (62, 63, 64, 65, 72, 73, 74, 75) aufweist, die sich von dem zentralen Körper (61, 71) verzweigen und sich parallel zueinander erstrecken und voneinander beabstandet sind, wobei der Schlitz (F) durch den Raum ausgebildet ist, der zwischen den Armen (62, 63, 64, 65, 72, 73, 74, 75) angeordnet ist.

3. Instrumente nach Anspruch 2, **dadurch gekennzeichnet, dass** sich die Arme (62, 63, 64, 65, 72, 73, 74, 75) der ersten und zweiten Führungseinheit (6, 7) auf eine gekrümmte Weise zur Annäherung der Krümmung eines Querschnitts der langen Knochen erstrecken.

4. Instrumente nach einem der vorhergehenden Ansprüche, **dadurch gekennzeichnet, dass** sie lineare Messmittel (8) für den Abstand zwischen der ersten und der zweiten Führungseinheit (6, 7) umfassen, die auf der Platte (3) verschiebbar in Eingriff stehen.

5. Instrumente nach Anspruch 4, **dadurch gekennzeichnet, dass** die linearen Messmittel ein millimetergenau skaliertes Lineal (8) umfassen.

6. Instrumente nach einem der vorhergehenden Ansprüche, **dadurch gekennzeichnet, dass** sie einen Fräser (10) zum Fräsen eines der Knochenstümpfe (4, 5) an dessen Endabschnitt, eine dritte Führungseinheit (13) zum Abstützen und verschiebbaren Führen des Fräsers (10) umfassen, wobei die dritte Führungseinheit (13) auf die Platte (3) eingreift.

7. Instrumente nach Anspruch 6, wobei der Fräser (10) einen Schaft (11) und einen Fräskopf (12) umfasst, der sich an einem Ende des Schafts (11) befindet, **dadurch gekennzeichnet, dass** die dritte Führungseinheit (13) ein hohlzylindrisches Element (14), das geeignet ist, um den Schaft (11) verschiebbar aufzunehmen und einen Greifkörper (15), der geeignet ist, um mit der Platte (3) zur stabilen Befestigung der dritten Führungseinheit (13) an der Platte einzugreifen, umfasst.

8. Instrumente nach Anspruch 6 oder 7, **dadurch gekennzeichnet, dass** der Fräskopf (12) eine kegelstumpfförmige Form aufweist, um jeweilige kegelstumpfförmige Hohlräume auf Endabschnitten (4a, 5a) der Stümpfe (4, 5) zu bilden.

9. Instrumente nach einem der vorhergehenden Ansprüche, umfassend eine Bearbeitungsvorrichtung (16) zum Bearbeiten der Knocheneinsätze (2), die zwischen zwei Stümpfen (4, 5) langer Knochen implantiert werden sollen, umfassend eine Stütz- und Befestigungsbasis (17) für einen zu bearbeitenden Einsatz (2), mindestens eine aufrechte Säule (18, 19), die vertikal aus der Basis (17) herausragt, ein Werkzeug (23) zum Bearbeiten des Einsatzes (2) und Führungsmittel (25, 27) für das Werkzeug (23), die von der aufrechten Säule (18, 19) abgestützt sind.

10. Instrumente nach Anspruch 9, **dadurch gekennzeichnet, dass** das Werkzeug zum Bearbeiten des Einsatzes (2) ein Fräser (23) ist, der zum Bilden von jeweiligen kegelstumpfförmigen Enden (2a, 2b) auf dem Einsatz (2) geformt ist.

## Revendications

1. Instruments permettant d'implanter des inserts d'os (2) entre deux moignons osseux (4, 5) d'os longs, comprenant :
- une plaque métallique (3) se prolongeant longitudinalement selon une direction prédéterminée (D) et adaptée pour relier les deux moignons osseux (4, 5) susmentionnés ;
- une première unité de guidage (6) servant à couper une partie d'extrémité d'un premier (4) desdits deux moignons, ladite première unité de guidage (6) étant configurée pour se mettre en prise de manière coulissante avec ladite plaque métallique (3) ;
- une seconde unité de guidage (7) servant à couper une partie d'extrémité d'un second (5) desdits deux moignons, ladite seconde unité de guidage (7) se mettant en prise de manière coulissante avec ladite plaque (3), chacune desdites première et seconde unités de guidage (6, 7) comportant une fente respective (F) servant à loger et à guider une lame de coupe, ladite fente (F) étant configurée pour guider une lame lorsqu'elle coupe ladite partie d'extrémité desdits moignons (4, 5) définissant un plan de coupe (P6, P7) se prolongeant perpendiculairement à ladite direction prédéterminée (D) d'extension de ladite plaque (3).

2. Instruments selon la revendication 1, **caractérisés en ce que** chacune desdites première et deuxième unités de guidage (6, 7) comprend un corps central (61, 71) servant à se mettre en prise avec ladite plaque (3) et des bras (62, 63, 64, 65, 72, 73, 74, 75) se ramifiant à partir dudit corps central (61, 71) et se prolongeant parallèlement et de façon espacée les uns des autres, ladite fente (F) étant formée par l'espace interposé entre lesdits bras (62, 63, 64, 65, 72, 73, 74, 75).

3. Instruments selon la revendication 2, **caractérisés en ce que** lesdits bras (62, 63, 64, 65, 72, 73, 74, 75) desdites première et deuxième unités de guidage (6, 7) se prolongent de manière incurvée pour se rapprocher de la courbure d'une section transversale des os longs.

4. Instruments selon l'une quelconque des revendications précédentes, **caractérisés en ce qu'**ils comprennent des moyens de mesure (8) linéaires de la distance entre lesdites première et deuxième unités de guidage (6, 7) étant mises en prise de manière coulissante avec ladite plaque (3).

5. Instruments selon la revendication 4, **caractérisés en ce que** lesdits moyens de mesure linéaires comprennent une règle graduée (8) millimétrique.

6. Instruments selon l'une quelconque des revendications précédentes, **caractérisés en ce qu'**ils comprennent une fraise (10) servant à fraiser l'un desdits moignons d'os (4, 5) à sa dite partie d'extrémité, une troisième unité de guidage (13) servant à supporter et à guider de manière coulissante ladite fraise (10), ladite troisième unité de guidage (13) se mettant en prise avec ladite plaque (3).

7. Instruments selon la revendication 6, dans lesquels ladite fraise (10) comprend un arbre (11) et une tête de fraisage (12) étant située à une extrémité dudit arbre (11), **caractérisés en ce que** ladite troisième unité de guidage (13) comprend un élément cylindrique creux (14) adapté pour loger de manière coulissante ledit arbre (11), et un corps de préhension (15) adapté pour se mettre en prise avec ladite plaque (3) pour fixer de façon stable ladite troisième unité de guidage (13) à ladite plaque.

8. Instruments selon la revendication 6 ou 7, **caractérisés en ce que** ladite tête de fraisage (12) possède une forme tronconique, pour réaliser des cavités tronconiques respectives sur les parties d'extrémité (4a, 5a) desdits moignons (4, 5).

9. Instruments selon l'une quelconque des revendications précédentes, comprenant un dispositif d'usinage (16) servant à usiner lesdits inserts osseux (2) à implanter entre deux moignons (4, 5) d'os longs, comprenant une base de support et de fixation (17) pour un insert (2) à usiner, au moins un montant à colonne (18, 19) émergeant verticalement de ladite base (17), un outil (23) servant à usiner ledit insert (2) et des moyens de guidage (25, 27) pour ledit outil (23) étant supportés par ledit montant à colonne (18, 19).

10. Instruments selon la revendication 9, **caractérisés en ce que** ledit outil servant à l'usinage dudit insert (2) est une fraise (23) façonnée pour réaliser des extrémités tronconiques (2a, 2b) respectives sur ledit insert (2).
